# EUROPEAN PATENT APPLICATION

(11) **EP 3 951 388 A1**
(43) Date of publication of application: **09.02.2022**
(21) Application number: 20777972.9
(22) Date of filing: 22.05.2020
(51) Int. Cl.: G01N 33/53, G01N 33/543, G01N 33/569

(54) **HUMAN METAPNEUMOVIRUS DETECTION REAGENT**

(30) Priority: 26.03.2019 JP 2019058483
(71) Applicant: Tanaka Kikinzoku Kogyo K.K., Tokyo 100-6422 (JP)
(72) Inventor: MOCHIZUKI, Hiroko, Kanagawa 254-0076 (JP)
(74) Representative: Hoffmann Eitle
(86) International application number: PCT/IB2020/054869
(87) International publication number: WO 2020/194285

(57) **Abstract**

The purpose of the present invention is to provide a detection reagent capable of detecting human metapneumovirus in a test specimen with excellent sensitivity. The present invention pertains to: a human metapneumovirus detection reagent characterized by containing an antibody that recognizes the matrix protein of human metapneumovirus; and an immunoassay method for human metapneumovirus whereby human metapneumovirus is detected in a specimen by means of an antibody that recognizes the matrix protein of human metapneumovirus.

SEQUENCE LISTING

<110>  TANAKA KIKINZOKU KOGYO K.K.

<120>  Reagent for detection of human metapneumovirus

<130>  W529485

<150>  JP2019-058483
<151>  2019-03-26

<160>  7

<170>  PatentIn version 3.5

<210>  1
<211>  254
<212>  PRT
<213>  human metapneumovirus

<400>  1

Met Glu Ser Tyr Leu Val Asp Thr Tyr Gln Gly Ile Pro Tyr Thr Ala
1               5                   10                  15

Ala Val Gln Val Asp Leu Val Glu Lys Asp Leu Leu Pro Ala Ser Leu
            20                  25                  30

Thr Ile Trp Phe Pro Leu Phe Gln Ala Asn Thr Pro Pro Ala Val Leu
        35                  40                  45

Leu Asp Gln Leu Lys Thr Leu Thr Ile Thr Thr Leu Tyr Ala Ala Ser
    50                  55                  60

Gln Asn Gly Pro Ile Leu Lys Val Asn Ala Ser Ala Gln Gly Ala Ala
65                  70                  75                  80

Met Ser Val Leu Pro Lys Lys Phe Glu Val Asn Ala Thr Val Ala Leu
                85                  90                  95

Asp Glu Tyr Ser Lys Leu Asp Phe Asp Lys Leu Thr Val Cys Glu Val
            100                 105                 110

Lys Thr Val Tyr Leu Thr Thr Met Lys Pro Tyr Gly Met Val Ser Lys
        115                 120                 125

Phe Val Ser Ser Ala Lys Ser Val Gly Lys Lys Thr His Asp Leu Ile
    130                 135                 140

Ala Leu Cys Asp Phe Met Asp Leu Glu Lys Asn Ile Pro Val Thr Ile
145                 150                 155                 160

Pro Ala Phe Ile Lys Ser Val Ser Ile Lys Glu Ser Glu Ser Ala Thr
                165                 170                 175

Val Glu Ala Ala Ile Ser Ser Glu Ala Asp Gln Ala Leu Thr Gln Ala
            180                 185                 190

Lys Ile Ala Pro Tyr Ala Gly Leu Ile Met Ile Met Thr Met Asn Asn
        195                 200                 205

Pro Lys Gly Ile Phe Lys Lys Leu Gly Ala Gly Thr Gln Val Ile Val
    210                 215                 220

Glu Leu Gly Ala Tyr Val Gln Ala Glu Ser Ile Ser Lys Ile Cys Lys
225                 230                 235                 240

Thr Trp Ser His Gln Gly Thr Arg Tyr Val Leu Lys Ser Arg
                245                 250

<210>  2
<211>  30
<212>  PRT
<213>  human metapneumovirus

<400>  2

Phe Met Asp Leu Glu Lys Asn Ile Pro Val Thr Ile Pro Ala Phe Ile
1               5                   10                  15

Lys Ser Val Ser Ile Lys Glu Ser Glu Ser Ala Thr Val Glu
            20                  25                  30

<210>  3
<211>  25
<212>  PRT
<213>  human metapneumovirus

<400>  3

Val Asp Thr Tyr Gln Gly Ile Pro Tyr Thr Ala Ala Val Gln Val Asp
1               5                   10                  15

Leu Val Glu Lys Asp Leu Leu Pro Ala
            20                  25

<210> 4
<211> 31
<212> PRT
<213> human metapneumovirus

<400> 4

Gln Ala Asn Thr Pro Pro Ala Val Leu Leu Asp Gln Leu Lys Thr Leu
1               5                   10                  15

Thr Ile Thr Thr Leu Tyr Ala Ala Ser Gln Asn Gly Pro Ile Leu
            20                  25                  30

<210> 5
<211> 35
<212> PRT
<213> human metapneumovirus

<400> 5

Ser Ala Gln Gly Ala Ala Met Ser Val Leu Pro Lys Lys Phe Glu Val
1               5                   10                  15

Asn Ala Thr Val Ala Leu Asp Glu Tyr Ser Lys Leu Asp Phe Asp Lys
            20                  25                  30

Leu Thr Val
        35

<210> 6
<211> 19
<212> PRT
<213> human metapneumovirus

<400> 6

Tyr Gly Met Val Ser Lys Phe Val Ser Ser Ala Lys Ser Val Gly Lys
1               5                   10                  15

Lys Thr His

<210> 7
<211> 36
<212> PRT
<213> human metapneumovirus

<400> 7

Glu Ala Asp Gln Ala Leu Thr Gln Ala Lys Ile Ala Pro Tyr Ala Gly
1               5                   10                  15

Leu Ile Met Ile Met Thr Met Asn Asn Pro Lys Gly Ile Phe Lys Lys
            20                  25                  30

Leu Gly Ala Gly
        35

## Description

### TECHNICAL FIELD

The present invention relates to a reagent for detecting human metapneumovirus, an immunological assay for human metapneumovirus and an immunochromatography analysis device and an immunochromatography analysis kit for detecting human metapneumovirus.

### BACKGROUND ART

Human metapneumovirus (sometimes simply referred to as hMPV below) is an RNA virus belonging to the genus *Metapneumovirus*, the subfamily *Pneumovirinae*, the family *Paramyxoviridae.*

hMPV is considered to be a virus causing acute respiratory infections mainly among children. The clinical symptoms are similar to those of RS virus infection, and upper respiratory tract inflammation and lower respiratory tract inflammation such as bronchitis and pneumonia are caused.

Viral isolation of hMPV has been considered to be relatively difficult, and genetic examination methods have been widely used for detecting hMPV (for example, NPL 1).

Recently, a diagnostic kit using immunochromatography assay has been developed to enable more simple and rapid detection of hMPV. The diagnostic kit uses an antibody to the N protein (nucleoprotein) or the F protein (fusion protein) of hMPV, which are on the surface of the virus particle and in which the mutation rate is low, for detecting hMPV (NPL 2).

### CITATION LIST

### NON PATENT LITERATURE

NPL 1: "A newly discovered human pneumovirus isolated from young children with respiratory tract disease" nature medicine 2001 Jun;7(6):719-24.
NPL 2: Modern Media, Vol. 60, No. 5, 2014, New Test Methods, A rapid human metapneumovirus (hMPV) antigen detection kit -Immunochromatography assay for the detection of hMPV covered by health insurance-

### SUMMARY OF INVENTION

### TECHNICAL PROBLEM

According to the examination of the present inventor, the sensitivity to actual analytes collected from individuals infected with hMPV of the diagnostic kit using an antibody to the N protein or the F protein of hMPV was not sufficient, and the diagnostic kit still had a room for improvement as a method for testing hMPV

Thus, to provide a detection reagent which can detect hMPV with an excellent sensitivity even when an actual analyte is used as an analyte sample, the present inventor has examined a new target subject which could be used instead of the N protein or the F protein of hMPV As a result of intensive studies, the present inventor has focused on the matrix protein of hMPV as the new target subject. The inventor has found that hMPV can be detected with a high sensitivity using an antibody which recognizes the protein and thus has completed the invention.

### SOLUTION TO PROBLEM

Accordingly, the invention is as follows.
1. A human metapneumovirus detection reagent characterized by containing an antibody which recognizes the matrix protein of human metapneumovirus.
2. The human metapneumovirus detection reagent according to 1 above, wherein the antibody recognizes the amino acid sequence of the 149-178th residues of the matrix protein of human metapneumovirus having the amino acid sequence of SEQ ID NO: 1.
3. An immunological assay for human metapneumovirus including detecting human metapneumovirus in an analyte with an antibody which recognizes the matrix protein of human metapneumovirus.
4. The immunological assay according to 3 above, wherein the immunological assay is enzyme immunoassay, agglutination assay or immunochromatography assay.
5. The immunological assay according to 4 above,
   wherein the immunological assay is immunochromatography assay,
   wherein the immunochromatography assay includes
   a step of bringing a labeled antibody in which a labeling substance is bound to an antibody which recognizes the matrix protein of human metapneumovirus into contact with the human metapneumovirus in the analyte and thus forming a complex of the labeled antibody and the human metapneumovirus, and
   a step of detecting the human metapneumovirus in the complex with a detection antibody which recognizes the matrix protein of human metapneumovirus.
6. The immunological assay according to 5 above, wherein the labeled antibody recognizes the amino acid sequence of the 149-178th residues of the matrix protein of human metapneumovirus having the amino acid sequence of SEQ ID NO: 1.
7. The immunological assay according to 5 or 6 above, wherein the detection antibody recognizes the three-dimensional structure of the matrix protein of human metapneumovirus.
8. An immunochromatography analysis device for detecting human metapneumovirus including a sample application part, a labeling substance-holding part, a chromatography medium part having a detection part and an absorption part,
   wherein the labeling substance-holding part and the detection part contain an antibody which recognizes the matrix protein of human metapneumovirus.
9. The immunochromatography analysis device according to 8 above, wherein the antibody contained in the labeling substance-holding part recognizes the amino acid sequence of the 149-178th residues of the matrix protein of human metapneumovirus having the amino acid sequence of SEQ ID NO: 1.
10. The immunochromatography analysis device according to 8 or 9 above, wherein the antibody contained in the detection part recognizes the three-dimensional structure of the matrix protein of human metapneumovirus.
11. An immunochromatography analysis kit including the immunochromatography analysis device according to any one of 8 to 10 above and an analyte dilution solution for diluting an analyte.

### ADVANTAGEOUS EFFECTS OF INVENTION

According to an embodiment of the invention, human metapneumovirus can be detected with a high sensitivity using an antibody which recognizes the matrix protein of human metapneumovirus. Moreover, according to an embodiment of the invention, human metapneumovirus can be detected rapidly and simply. Furthermore, according to an embodiment of the invention, hMPV can be detected specifically without causing a cross-reaction with a virus having a high homology to hMPV such as RSV

### BRIEF DESCRIPTION OF DRAWINGS

[Fig. 1] Fig. 1 is a cross section for explaining the structure of the immunochromatography analysis device of an embodiment of the invention.
[Fig. 2] Fig. 2 is a graph showing the results (absorbances) of an ELISA test conducted for examining the amino acid sequences of the matrix protein of hMPV which the antibodies produced in the Examples recognize.
[Fig. 3] Fig. 3 is a graph showing the results (S/N ratios) of an ELISA test conducted for examining the amino acid sequences of the matrix protein of hMPV which the antibodies produced in the Examples recognize.
[Fig. 4] Figs. 4(a) and (b) are pictures showing the results of SDS-PAGE and western blotting conducted for examining whether the antibodies produced in the Examples recognize the three-dimensional structure of the matrix protein of hMPV The arrows in the figures show the bands of about 36 kDa corresponding to the whole length hMPV matrix protein.
[Fig. 5] Fig. 5 is a graph showing the results of evaluation of detection sensitivities using the immunochromatography analysis kits of Example 1.
[Fig. 6] Fig. 6 is a graph showing the results of evaluation of detection sensitivities using the immunochromatography analysis kits of Example 2.
[Fig. 7] Fig. 7 is a graph showing the results of evaluation of cross-reactivities using the immunochromatography analysis kits of Example 1.

### DESCRIPTION OF EMBODIMENTS

Embodiments for carrying out the invention are explained below.

In this specification, that the antibody in the invention "recognizes" the matrix protein of hMPV means that the antibody substantially undergoes the antigen-antibody reaction with the matrix protein of hMPV. Moreover, that the antibody in the invention "recognizes" a specific amino acid sequence of the matrix protein of hMPV means that the antibody substantially undergoes the antigen-antibody reaction with the whole or a part of the amino acid sequence.

Whether the antibody in the invention substantially undergoes the antigen-antibody reaction with the matrix protein of hMPV or the whole or a part of a specific amino acid sequence thereof can be examined by a known immunoassay. When immunoassays are classified by the method of immunoassay, examples thereof include sandwich assay, competitive assay, agglutination assay and the like. When immunoassays are classified by the label used, examples thereof include fluorescence assay, enzyme assay, radiation assay and the like. The antigen-antibody reaction can be identified by any of the immunoassays.

Here, "substantially undergoes the antigen-antibody reaction" means the antigen-antibody reaction at a level detectable with the immunoassay and means that a reaction which is at a clearly low degree and which is not a specific reaction is excluded even when the reaction is at a detectable level.

The absorbance of the antibody in the invention which is obtained by measuring the absorbance by the ELISA test described in Reference Example 1 described below and subtracting the blank value is preferably 0.1 Abs or more, more preferably 0.15 Abs or more, further preferably 0.2 Abs or more.

Moreover, the S/N ratio of the antibody in the invention which is obtained by measuring the absorbance by an ELISA test using the target protein that the antibody recognizes or a peptide having the amino acid sequence as an antigen and dividing the absorbance by the absorbance of the blank is preferably 1.4 or more, more preferably 1.5 or more, further preferably 1.6 or more.

The analyte which can be applied to the invention is not particularly limited as long as human metapneumovirus may be contained. Examples of the analyte include blood samples such as blood, plasma and serum, urine, saliva, spinal fluid, sweat, tears, amniotic fluid, nipple discharge, nasal discharge, phlegm, nasal aspirate, nasal swab, nose blow sample, pharyngeal swab, skin effusion, extracts from tissues, cells and feces and the like, which are actual analytes of individuals infected with human metapneumovirus. Of these examples, nasal aspirate, nasal swab, nose blow sample and pharyngeal swab are preferable in view of the rapid diagnosis and the improvement of the detection sensitivity.

### [Detection Reagent]

The human metapneumovirus detection reagent of the invention is characterized by containing an antibody which recognizes the matrix protein of hMPV.

The matrix protein of hMPV is composed of the 254 amino acids shown in SEQ ID NO: 1 below.

(SEQ ID NO: 1)
MESYLVDTYQGIPYTAAVQVDLVEKDLLPASLTIWFPLFQANTPPAVLLDQLKTLTITT
LYAASQNGPILKVNASAQGAAMSVLPKKFEVNATVALDEYSKLDFDKLTVCEVKTVY
LTTMKPYGMVSKFVSSAKSVGKKTHDLIALCDFMDLEKNIPVTIPAFIKSVSIKESESA
TVEAAISSEADQALTQAKIAPYAGLIMIMTMNNPKGIFKKLGAGTQVIVELGAYVQAE
SISKICKTWSHQGTRYVLKSR

The matrix protein of hMPV which the antibody in the invention recognizes may be a natural protein isolated from the virus or a recombinant protein produced based on a known nucleotide sequence of a gene encoding the matrix protein. Moreover, the matrix protein of hMPV may be one which has been isolated/purified from the components constituting the virus or unpurified one. When the matrix protein is not isolated, the matrix protein may be matrix protein derived from the virus which has been treated with a surfactant so that the matrix protein and the antibody come into contact easily.

In the invention, using an antibody which recognizes particularly the matrix protein, of various proteins that hMPV has, hMPV can be detected with a high sensitivity even from an actual analyte.

The detection reagent of the invention preferably contains an antibody which recognizes the amino acid sequence of the 149-178th residues of the matrix protein of hMPV having the amino acid sequence of SEQ ID NO: 1 because the detection sensitivity can be further improved.

The absorbance of the antibody which recognizes the amino acid sequence of the 149-178th residues which is obtained by subtracting the blank value from the absorbance measured using peptide 5 as an antigen in the ELISA test described in Reference Example 1 described below is preferably 0.1 Abs or more, more preferably 0.15 Abs or more, further preferably 0.2 Abs or more.

The S/N ratio of the antibody which recognizes the amino acid sequence of the 149-178th residues which is obtained by measuring the absorbance by an ELISA test using peptide 5 as an antigen and dividing the absorbance by the absorbance of the blank is preferably 1.4 or more, more preferably 1.5 or more, further preferably 1.6 or more.

The amino acid sequence of the 149-178th residues is the sequence of SEQ ID NO: 2 below.
(SEQ ID NO: 2)
FMDLEKNIPVTIPAFIKSVSIKESESATVE

Examples of the antibody in the invention include natural antibodies such as polyclonal antibodies and monoclonal antibodies, chimeric antibodies, humanized antibodies and single-chain antibodies that can be produced using gene recombination, human antibodies that can be produced using a human antibody-producing transgenic animal or the like, antibodies produced by phage display and fragments thereof with binding capacity. A monoclonal antibody is preferable in view of the detection sensitivity.

Examples of the method for producing the antibody in the invention are explained below.

Regarding the antibody-producing animal species, for example, human, mouse, rat, rabbit, goat, horse and the like can be used. The immunoglobulin may be any of IgG, IgM, IgA, IgE and IgD.

In an embodiment of the method for producing the antibody in the invention, the peptide of the matrix protein of hMPV as the immunogen can be produced by a known general production method. For example, a peptide of the matrix protein that is extracted and purified from hMPV, a peptide of hMPV matrix protein that is obtained by expressing cloned gene of the matrix protein in a host such as *Escherichia coli* by genetic engineering and extracting and purifying the peptide or a polypeptide which composes a part of a peptide of hMPV matrix protein can be used as the immunogen.

With respect to a monoclonal antibody, according to a general method, after hybridizing spleen cells of a mouse immunized with the immunogen and myeloma cells, a hybridoma that produces the target antibody is selected, and the monoclonal antibody produced by the hybridoma is obtained [for example, see the Köhler and Milstein's technique (Nature 256 (1975) 495-497)]. A polyclonal antibody is obtained by separating the target antibody from the antiserum obtained by immunizing an animal for production (for example, human, mouse, rat, rabbit, goat, horse or the like) with the immunogen according to a general method.

Screening to obtain the hybridoma clone that produces a monoclonal antibody can be conducted by culturing hybridomas for example in a microtiter plate and measuring the reactivities of the culture supernatants of the wells in which the growth is observed with the immunogen by enzyme immunoassay such as ELISA.

The hybridoma can be cultured using a medium (for example, DMEM containing 10% fetal bovine serum), and the supernatant of the culture solution obtained by centrifugation can be used as a monoclonal antibody solution. Also, ascites can be caused by injecting the hybridoma into the abdominal cavity of the origin animal, and the obtained ascites can be used as a monoclonal antibody solution. The monoclonal antibody is preferably isolated and/or purified.

An antibody which recognizes the matrix protein of hMPV can be produced by culturing the hybridoma in a medium which is usually used for cell culture and collecting from the culture supernatant. Moreover, the antibody can also be prepared by accumulating ascites by injecting the hybridoma into the abdominal cavity of the origin animal and collecting from the ascites.

Of antibodies which recognize the matrix protein of hMPV, an antibody which recognizes the amino acid sequence of the 149-178th residues (SEQ ID NO: 2) of the matrix protein of hMPV having the amino acid sequence of SEQ ID NO: 1 can be produced as follows. That is, antibodies which recognize the matrix protein of hMPV are produced as described above and then subjected to ELISA method, western blotting or the like using a peptide fragment corresponding to the amino acid sequence of SEQ ID NO: 2, and thus a hybridoma which produces an antibody exhibiting certain reactivity with the amino acid sequence of SEQ ID NO: 2 is selected from the hybridomas that produce antibodies recognizing the matrix protein of hMPV. In this manner, an antibody which recognizes SEQ ID NO: 2 can be obtained and used.

The detection reagent of the invention can be preferably used when hMPV in a biological sample is detected by enzyme immunoassay (EIA method, ELISA method or the like), agglutination assay, immunochromatography assay or the like.

For example, in the case of ELISA method, detection can be made as follows. First, the reagent of the invention is dispensed to a well of a microplate, and the well bottom is sensitized by the antibody which recognizes the matrix protein of hMPV Next, an analyte sample is added to the well of the microplate, and a complex of the hMPV contained in the sample and the antibody is formed. Then, by adding a labeled antibody obtained by labeling an antibody which recognizes the matrix protein of hMPV to the well, the labeled antibody is bound to the complex. Based on the labeling with the labeled antibody, hMPV can be detected.

The detection reagent of the invention can contain a component other than the antibody depending on the use of the reagent, namely the assay method conducted using the reagent.

Examples of the other component include a buffer solution, a surfactant, a salt, a protein, an antiseptic and the like. In the case of ELISA method, a chromogenic substrate or the like is also included. In agglutination assay, a thickener or the like is also included.

Examples of the buffer solution include lactate buffer, citrate buffer, acetate buffer, succinate buffer, phthalate buffer, phosphate buffer, triethanolamine buffer, diethanolamine buffer, lysine buffer, barbiturate buffer, imidazole buffer, malate buffer, oxalate buffer, glycine buffer, borate buffer, carbonate buffer, glycine buffer, Good's buffers and the like.

Examples of the surfactant include nonionic surfactants, anionic surfactants, cationic surfactants and the like.

Examples of the salt include sodium chloride, potassium chloride and the like.

Examples of the protein include bovine serum albumin (BSA), fetal bovine serum (FBS), casein, Block Ace (manufactured by Dainippon Pharma Co., Ltd.) and the like.

Examples of the antiseptic include sodium azide, isothiazoline, antibiotics (streptomycin, penicillin, gentamicin and the like), BioAce, ProClin 300, Proxel GXL and the like.

Examples of the chromogenic substrate include 3,3',5,5'-tetramethylbenzidine (TMB), *o*-phenylenediamine (OPD), *p*-nitrophenyl phosphate (pNPP), *o*-nitrophenyl-(β-D-galactopyranoside and the like.

Examples of the thickener include gelatin, agar, glycerol, starch, alginates, dextrin, carrageenan, chitosan and the like.

### [Immunological Assay]

The immunological assay of the invention is characterized by detecting hMPV in an analyte with an antibody which recognizes the matrix protein of hMPV.

In the assay of the invention, as the antibody which recognizes the matrix protein of hMPV, the antibody used for the detection reagent of the invention can be used.

Examples of specific embodiments of the assay of the invention include enzyme immunoassay (EIA method or ELISA method), agglutination assay or immunochromatography assay. Of the examples, immunochromatography assay is preferable because the detection can be made simply and rapidly with a high sensitivity.

The immunochromatography assay as the immunological assay of the invention preferably includes at least (1) a step of bringing a labeled antibody in which a labeling substance is bound to an antibody which recognizes the matrix protein of hMPV into contact with the hMPV in the analyte and thus forming a complex of the labeled antibody and the hMPV and (2) a step of detecting the hMPV in the complex with a detection antibody which recognizes the matrix protein of hMPV

The immunochromatography assay as the immunological assay of the invention is specifically explained below based on an immunochromatography analysis device used for the assay.

In an embodiment, the immunochromatography assay of the invention includes the following steps (1) to (4) and detects the hMPV contained in an analyte using an immunochromatography analysis device.

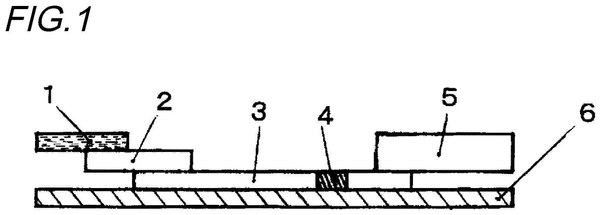

The details of the immunochromatography analysis device in an embodiment are as described below, but the immunochromatography analysis device is composed of a sample application part 1, a labeling substance-holding part 2, a chromatography medium part 3, a detection part 4, an absorption part 5 and a backing sheet 6 as shown in Fig. 1. In the device, "immobilize" means that an antibody is arranged on a carrier such as a membrane in a manner that the antibody does not move, and "hold" means that an object is arranged in a manner that the object can move in a carrier such as a membrane or on the surface thereof.

Step (1): a step of applying an analyte-containing solution obtained by diluting the analyte with an analyte dilution solution as a sample to the sample application part
Step (2): a step of bringing a labeled antibody in which a labeling substance is bound to an antibody recognizing the matrix protein of hMPV and which is held in the labeling substance-holding part into contact with the hMPV in the analyte and thus forming a complex of the labeled antibody and the hMPV
Step (3): a step of developing the complex as a mobile phase on the chromatography medium part
Step (4): a step of detecting the hMPV in the developed complex with a detection antibody which recognizes the matrix protein of hMPV and which is immobilized on the detection part

Each step is explained below.

Step (1): a step of applying an analyte-containing solution obtained by diluting the analyte with an analyte dilution solution as a sample to the sample application part

First, an analyte-containing solution is preferably obtained by adjusting or diluting the analyte with an analyte dilution solution to a concentration at which the analyte moves smoothly in the medium of the device without deteriorating the measurement accuracy.

Those described below can be used as the analyte dilution solution.

Secondly, a certain amount (usually 0.1 ml to 2 ml) of the analyte-containing solution is dropped as a sample onto the sample application part 1. When the sample is dropped to the sample application part 1, the sample starts to move in the sample application part 1.

Step (2): a step of bringing a labeled antibody in which a labeling substance is bound to an antibody recognizing the matrix protein of hMPV and which is held in the labeling substance-holding part into contact with the hMPV in the analyte and thus forming a complex of the labeled antibody and the hMPV

The step (2) is a step for transferring the sample applied to the sample application part in the step (1) to the labeling substance-holding part 2, bringing a labeled antibody in which a labeling substance is bound to an antibody recognizing the matrix protein of hMPV and which is held in the labeling substance-holding part 2 into contact with the hMPV, which is the substance to be detected, in the analyte, thus causing the labeled antibody to recognize the hMPV and forming a complex of the both. Those described below can be used as the labeling substance.

Step (3): a step of developing the complex as a mobile phase on the chromatography medium part

The step (3) is a step in which the complex formed when the hMPV, which is the substance to be detected, has been brought into contact with and recognized by the labeled antibody in the labeling substance-holding part 2 in the step (2) is caused to pass through on the chromatography medium part 3 as a mobile phase.

Step (4): a step of detecting the hMPV in the developed complex with a detection antibody which recognizes the matrix protein of hMPV and which is immobilized on the detection part

The step (4) is a step in which the hMPV in the analyte that has passed through on the chromatography medium part 3 as the mobile phase specifically reacts and binds by a specific antigen-antibody binding reaction in a manner that the hMPV is sandwiched between the detection antibody which recognizes the matrix protein of hMPV and which is immobilized on the detection part 4 and the labeled antibody to which the labeling substance has bound in the step (2), resulting in the coloration of the detection part 4.

When hMPV, which is the substance to be detected, is absent, the labeling substance dissolved in the water content of the sample does not cause the specific binding reaction even when the labeling substance passes through the detection part on the chromatography medium part 3, and thus the detection part 4 is not colored.

At the end, the water content in the analyte-containing solution moves to the absorption part 5.

The labeled antibody preferably recognizes the amino acid sequence of the 149-178th residues of the matrix protein of hMPV having the amino acid sequence of SEQ ID NO: 1. When the antibody is used, the detection sensitivity can be further improved. As the antibody which recognizes the amino acid sequence of the 149-178th residues of the matrix protein of hMPV having the amino acid sequence of SEQ ID NO: 1, those described above can be used.

The detection antibody preferably recognizes the three-dimensional structure of the matrix protein of hMPV. When the antibody is used, the detection sensitivity can be further improved. This can be speculated to be because the labeled antibody first recognizes and binds to the primary structure (the peptide sequence) of the matrix protein, which is the subject to be detected, and thus the three-dimensional structure of the matrix protein is stabilized and is more easily supplemented by the antibody which recognizes the three-dimensional structure in the detection part. This can be also speculated to be because, when the detection antibody recognizes the three-dimensional structure, the detection antibody has easier access also to the matrix protein to which the labeled antibody has bound compared to the case in which the detection antibody recognizes the primary structure (the peptide sequence), and the site on the matrix protein surface recognized by the detection antibody is not limited.

The matrix protein of hMPV that the antibody recognizes may be matrix protein which maintains a sufficient three-dimensional structure for maintaining the antigen-antibody reaction with at least a certain antibody, of the three-dimensional structure of naturally existing matrix protein of hMPV, and those which no longer maintain the substantial antigen-antibody reaction of the matrix protein to the antibody after the three-dimensional structure of naturally existing matrix protein has been destroyed by SDS-PAGE or the like are excluded.

The antibody may be an antibody which does not substantially undergo the antigen-antibody reaction with the protein in which the three-dimensional structure of the site for the antigen-antibody reaction in naturally existing matrix protein of hMPV has been destroyed. An example of such an antibody is an antibody which does not undergo the antigen-antibody reaction with the whole length matrix protein of hMPV separated by SDS-PAGE by western blotting. Here, that an antibody does not undergo the antigen-antibody reaction by western blotting means that the antibody does not undergo the antigen-antibody reaction at a detectable level under the standard conditions for the antibody concentration, the antigen concentration, the substrate concentration, the reaction period or the like in western blotting.

"SDS-PAGE" is a separation/analysis method of a protein which is commonly used in the technical field to which the invention pertains and can be typically conducted according to the method of Laemmli, U.K. (Nature, 227: 680-685 (1970)) although SDS-PAGE is not limited to the method.

Specifically, for example, SDS-PAGE can be conducted by the following procedures. First, a resolving gel containing polyacrylamide at a concentration of 10 to 15% is cast between gel plates, and a stacking gel containing 3 to 5% polyacrylamide is cast on top of the resolving gel. The produced gels are mount in a slab electrophoresis apparatus. To a solution containing the matrix protein of hMPV, an equivalent amount of 2× sample buffer (125 mM Tris-HCl, 20% glycerol, 2% SDS, 2% 2-mercaptoethanol, 0.001% bromophenol blue, pH 6.8) is added, and the mixture is heat-treated at 100°C for 5 to 10 minutes. The sample for electrophoresis is thus prepared. The sample for electrophoresis and a commercial molecular weight marker are loaded in the lanes created in the stacking gel, and the electrophoresis is conducted using an electrophoresis buffer (192 mM glycine, 0.1% SDS, 24 mM Tris, pH 8.3) with a constant current of 20 mA for 30 to 90 minutes. The whole length matrix protein of hMPV separated by SDS-PAGE can be obtained in a band corresponding to the molecular weight of about 36 kDa in the resolving gel.

The solution containing the matrix protein of hMPV used for SDS-PAGE is not limited as long as the solution contains the matrix protein of hMPV in an amount sufficient for the antigen-antibody reaction with the antibody in the eventual western blotting (for example, 1 to 2 mg), and the solution may be purified but does not have to be purified in terms of the matrix protein. Examples of the solution containing the matrix protein of hMPV include a hMPV suspension, a commercial hMPV vaccine, a recombinant hMPV matrix protein solution and the like.

SDS in the 2× sample buffer used for SDS-PAGE can be used with an appropriate modification in a concentration range of 0.5 to 5% by weight depending on the amount of the matrix protein of hMPV considering that the amount of bound SDS is about 1.2 to 1.5 based on the protein amount considered as 1. Moreover, 2-mercaptoethanol in the 2× sample buffer functions as a reducing agent which cuts the disulfide bonds in the matrix protein of hMPV and may be used with an appropriate modification in a concentration range of 1 to 10% by weight. A reducing agent composed of another substance such as dithiothreitol (DTT) and tris(2-carboxyethyl) phosphine hydrochroride (TCEP-HCl) can also be used.

"Western blotting" can be conducted by transferring the whole length matrix protein of hMPV separated by the SDS-PAGE onto a polyvinylidene difluoride (PVDF) membrane according to, for example, the method of Towbin H. et al. [Proc.Natl.Acad.Sci. U.S.A., 76: 4350-4354 (1979)] but is not limited to this method.

Specifically, for example, a PVDF membrane is immersed in 100% methanol for 10 seconds and in an electrode buffer for transfer (192 mM glycine, 5% methanol, 25 mM Tris-HCl, pH 8.3) for 30 minutes and used for the transfer. The transfer apparatus is assembled by placing a filter paper, the PVDF membrane, the gel after the completion of SDS-PAGE and a filter paper in this order on the anode plate and fixing the cathode plate on top. The filter papers are immersed in advance in the electrode buffer for transfer for two to three minutes.

The transfer is conducted with a constant current of 1.9 mA/cm² for 60 to 90 minutes. The PVDF membrane after the completion of the transfer is incubated in a blocking solution (0.5% BSA, 10 mM Tris-HCl, 140 mM NaCl, 0.01% Tween 20, pH 7.5) at room temperature for 60 minutes for blocking operation. After the completion of the blocking, the membrane is incubated and washed twice with a wash buffer (10 mM Tris-HCl, 140 mM NaCl, 0.01% Tween 20, pH 7.5) for five minutes and incubated and reacted using the detection antibody as a primary antibody at room temperature for 90 minutes.

After the completion of the reaction with the primary antibody, the membrane is incubated and washed twice with the wash buffer for five minutes and incubated and reacted using an antibody which specifically reacts with the primary antibody and which is labeled with a labeling substance such as an enzyme, a fluorescent substance and a radioisotope as a secondary antibody at room temperature for 60 minutes. After the completion of the reaction with the secondary antibody, the membrane is incubated and washed twice with the wash buffer for five minutes, and then the primary antibody bound to the matrix protein of hMPV transferred to the PVDF membrane is visualized using the properties of the labeling substance for the detection of western blotting.

That the detection antibody does not undergo the antigen-antibody reaction with the whole length matrix protein of hMPV separated by SDS-PAGE by western blotting can be confirmed, for example using a commercial antibody which has been confirmed to undergo the antigen-antibody reaction with the matrix protein of hMPV by western blotting as a positive control antibody, using that the matrix protein of hMPV cannot be detected under the condition under which the positive control antibody undergoes the antigen-antibody reaction with the matrix protein of hMPV on a PVDF membrane and can detect the matrix protein as a standard.

The detection antibody is preferably an antibody which does not undergo the antigen-antibody reaction with the matrix protein of hMPV at the same antibody concentration as the antibody concentration at which the positive control antibody can detect the matrix protein of hMPV by western blotting to improve the detection sensitivity. The detection antibody is more preferably an antibody which does not react with the matrix protein of hMPV at the antibody concentration which is two times higher, further preferably an antibody which does not undergo the antigen-antibody reaction with the matrix protein of hMPV at an antibody concentration which is five times or 10 times higher.

The detection antibody is preferably an antibody which does not undergo the antigen-antibody reaction with the matrix protein of hMPV at the same concentration as the antigen concentration of the matrix protein of hMPV which the positive control antibody can detect by western blotting to improve the detection sensitivity. The detection antibody is more preferably an antibody which does not react at the antigen concentration which is two times higher, further preferably an antibody which does not undergo the antigen-antibody reaction with the matrix protein of hMPV at a concentration which is five times or 10 times higher.

The method for producing the antibody described above can be applied for producing the antibody which recognizes the three-dimensional structure of the matrix protein of hMPV, and the immunogen is preferably the matrix protein of hMPV which has not been treated with a sample buffer for SDS-PAGE containing a reducing agent, more preferably the matrix protein of hMPV which has not been treated with SDS, which is an anionic surfactant. Preferable examples of the immunogen include a suspension of hMPV in a buffer containing no anionic surfactant, the whole length matrix protein of hMPV and the like.

In the immunochromatography assay as the immunological assay of the invention, it is preferable to use an antibody which recognizes the amino acid sequence of the 149-178th residues of the matrix protein of hMPV having the amino acid sequence of SEQ ID NO: 1 as the labeled antibody and to use an antibody which recognizes the three-dimensional structure of the matrix protein of hMPV as the detection antibody, in order to improve the detection sensitivity. Reasons therefore are speculated to be as follows: when the combination of the labeled antibody and the detection antibody is as described above, the labeled antibody first recognizes and binds to the primary structure (the peptide sequence) of the matrix protein, which is the subject to be detected, and thus the three-dimensional structure of the matrix protein is stabilized and is more easily supplemented by the antibody which recognizes the three-dimensional structure in the detection part; and because the detection antibody recognizes the three-dimensional structure, the site on the matrix protein surface recognized by the detection antibody is not limited, and thus the detection antibody can more easily capture the matrix protein-labeled antibody complex.

### [Immunochromatography Analysis Device]

As shown in Fig. 1, the immunochromatography analysis device of the invention is composed of a sample application part 1, a labeling substance-holding part 2, a chromatography medium part 3, a detection part 4, an absorption part 5 and a backing sheet 6.

The sample application part 1 is a part in the immunochromatography analysis device to which a sample containing an analyte is applied. The sample application part 1 can be composed of a porous sheet having the properties of rapidly absorbing the sample but allowing the sample to move rapidly. Examples of the porous sheet include cellulose filter paper, glass fibers, polyurethane, polyacetate, cellulose acetate, nylon, cotton cloth and the like.

The labeling substance-holding part 2 is a part which holds a labeled antibody to which the labeling substance described below is bound. When the sample moves in the labeling substance-holding part 2, the labeled antibody binds to the substance to be detected in the analyte. The labeled antibody is an antibody which recognizes the matrix protein of hMPV. The labeled antibody preferably recognizes the amino acid sequence of the 149-178th residues of the matrix protein of hMPV having the amino acid sequence of SEQ ID NO: 1 to improve the detection sensitivity.

For the labeling substance-holding part 2, a membrane of glass fibers, cellulose or the like is usually used.

The amount of the labeled antibody in the labeling substance-holding part 2 is usually 0.05 µg/device to 0.5 µg/device, preferably 0.05 µg/device to 0.25 µg/device, more preferably 0.07 µg/device to 0.1 µg/device. The amount of the labeled antibody per unit area of the labeling substance-holding part 2 is usually 0.15 (µg/cm² to 1.5 µg/cm², preferably 0.15 µg/cm² to 1.0 µg/cm², more preferably 0.20 µg/cm² to 0.25 µg/cm². When the amount of the labeled antibody in the labeling substance-holding part 2 is in the range, an excellent detection sensitivity can be achieved.

An enzyme or the like is also generally used as the labeling substance for labeling an antibody in an immunochromatography analysis, but an insoluble carrier is preferably used as the labeling substance because the insoluble carrier is suitable for visually determining the presence of the substance to be detected. That is, in the invention, a labeled antibody which is labeled by sensitizing an antibody which recognizes the matrix protein of hMPV by an insoluble carrier is preferably used as the antibody contained in the labeling substance-holding part 2. In this regard, the means for sensitizing the antibody which recognizes the matrix protein of hMPV by the insoluble carrier may be in accordance with a known method.

As the insoluble carrier used as the labeling substance, particles of a metal such as gold, silver and platinum, particles of a metal oxide such as iron oxide, particles of a nonmetal such as sulfur, latex particles of a synthetic polymer or other insoluble carriers can be used. As described above, the insoluble carrier is a labeling substance which is suitable for visually determining the presence of the substance to be detected and preferably has a color to make the visual determination easy. Metal particles and metal oxide particles themselves have peculiar natural colors according to the particle diameter, and the colors can be used as labels.

The insoluble carrier used as the labeling substance is especially preferably gold particles because gold particles are simple to detect and do not easily cohere and because nonspecific color development is unlikely to occur. The average particle diameter of the gold particles is, for example, 10 nm to 250 nm, preferably 35 nm to 120 nm. The average particle diameter can be calculated by measuring the projected area circle equivalent diameters of 100 particles at random using projected pictures taken with a transmission electron microscope (TEM: manufactured by JEOL Ltd., JEM-2010) and calculating from the average. The amount of the gold particles in the labeling substance-holding part is, per unit area of the labeling substance-holding part, usually 0.006 (µg/cm² to 0.42 µg/cm², preferably 0.01 µg/cm² to 0.3 µg/cm², more preferably 0.01 µg/cm² to 0.2 µg/cm². This is because, by determining the amount in the range, the labeled particles can be developed while the particles are dispersed, and the recognition sites for the antibody are not inhibited, resulting in an increase in the sensitivity.

The chromatography medium part 3 is a part for development of chromatography. The chromatography medium part 3 is an inert membrane composed of a fine porous substance which causes a capillary phenomenon. Because the membranes do not have the property of reacting with the detection reagent or the immobilizing reagent used for chromatography or with the substance to be detected or the like and because the detection sensitivity improves, for example, nitrocellulose membranes and cellulose acetate membranes are preferable, and nitrocellulose membranes are further preferable. In this regard, cellulose membranes, nylon membranes and porous plastic clothes (for example, polyethylene, polypropylene or the like) can also be used.

The nitrocellulose membranes may be any nitrocellulose membranes as long as the membranes mainly contain nitrocellulose, and membranes containing nitrocellulose as the main material, such as a pure product or a nitrocellulose-mixed product, can be used.

The nitrocellulose membranes can also contain a substance which further enhances the capillary phenomenon. The substance is preferably a substance which weakens the surface tension of the membrane surface and attains hydrophilicity to enable rapid and accurate measurement. For example, a substance which has amphipathic action, like saccharides, derivatives of amino acids, fatty acid esters, various synthetic surfactants, alcohols or the like, and which does not affect the movement of the substance to be detected and does not affect the color development of the labeling substance is preferable.

The nitrocellulose membranes are porous and cause a capillary phenomenon. The indicator of the capillary phenomenon can be confirmed by measuring the speed of water absorption (water absorption time: capillary flow time). The speed of water absorption affects the detection sensitivity and the test time.

The form and the size of the chromatography medium part 3, which is typically any of the nitrocellulose membranes or the cellulose acetate membranes described above, are not particularly limited and may be any form and any size as long as they are appropriate for the actual operation and for the observation of the reaction results.

In order to further make the operation simpler, a support composed of plastic or the like is preferably provided on the back surface of the chromatography medium part 3. The properties and state of the support are not particularly limited. When the measurement results are observed by a visual evaluation, the support is preferably a support having a color which is not similar to the color achieved by the labeling substance, and the support is usually preferably colorless or white.

In order to prevent the deterioration of the analysis accuracy due to nonspecific adsorption on the chromatography medium part 3, the chromatography medium part 3 can be subjected to blocking treatment by a known method according to the need. For the blocking treatment, in general, a protein such as bovine serum albumin, skim milk, casein and gelatin are preferably used. After the blocking treatment, the chromatography medium part 3 may be washed with one or a combination of two or more of surfactants such as Tween 20, Triton X-100 and SDS according to the need.

The detection part 4 is formed at any position on the chromatography medium part 3 and contains an antibody which recognizes the matrix protein of hMPV as the detection antibody. The antibody which recognizes the three-dimensional structure of the matrix protein of hMPV described above is preferably contained to improve the detection sensitivity.

The antibody which recognizes the matrix protein of hMPV can be immobilized on the detection part 4 according to a general method.

In the detection part 4, the hMPV in the analyte that has passed through on the chromatography medium part as the mobile phase specifically binds in a manner that the hMPV is sandwiched between the detection antibody that is immobilized on the detection part 4 and the labeled antibody to which the labeling substance is bound.

The amount of the antibody which recognizes the matrix protein of hMPV contained in the detection part 4 is usually 0.05 µg/device to 5.0 µg/device, preferably 0.08 µg/device to 3.0 µg/device, more preferably 0.1 µg/device to 1.0 µg/device. The amount of the antibody which recognizes the matrix protein of hMPV per unit area of the detection part 4 is usually 0.1 µg/cm² to 50 µg/cm², preferably 0.8 µg/cm² to 30 µg/cm², more preferably 1 µg/cm² to 10 µg/cm². When the amount of the detection antibody in the detection part 4 is in the range, an excellent detection sensitivity can be achieved.

The absorption part 5 is provided at the end of the chromatography medium part 3 to absorb liquids such as the analyte and the development solution which have passed through the detection part 4. In the invention, for example, glass fibers, pulp, cellulose fibers or these nonwoven clothes to which a polymer such as acrylic polymers and a hydrophilic agent having an ethylene oxide group or the like have been added are used for the absorption part 5. Glass fibers are preferable because glass fibers can excellently absorb liquids.

The backing sheet 6 is a base material. One surface thereof is adhesive because an adhesive is applied on the surface or an adhesive tape is attached, and the sample application part 1, the labeling substance-holding part 2, the chromatography medium part 3, the detection part 4 and the absorption part 5 are partially or entirely closely adhered and provided on the adhesive surface. The base material is not particularly limited as long as the backing sheet 6 is not permeable or breathable with respect to the sample solution due to the adhesive.

The immunochromatography analysis device of the invention is usually subjected to drying treatment before being finished as a product. The drying temperature is, for example, 20°C to 50°C, and the drying time is 0.5 hours to 1 hour.

### [Immunochromatography Analysis Kit]

The immunochromatography analysis kit of the invention includes the immunochromatography analysis device and an analyte dilution solution for diluting and developing an analyte.

In the immunochromatography analysis kit of the invention, the analyte dilution solution can be used also as a development solution. Water is usually used as a solvent of the analyte dilution solution, and a buffer solution, a salt and a nonionic surfactant are contained. A kind or two or more kinds of a protein, a polymer compound (such as PVP), an ionic surfactant or a polyanion for, for example, promoting the antigen-antibody reaction or inhibiting a nonspecific reaction, an antibacterial agent, a chelating agent and the like may be further added.

When the analyte dilution solution is used as a development solution, the analyte and the development solution can be mixed in advance and then supplied/dropped as the sample to the sample application part for development, or the development solution may be supplied/dropped to the sample application part for development after supplying/dropping the sample containing the analyte to the sample application part in advance.

### EXAMPLES

The invention is further explained below with Examples, but the invention is not limited to the following Examples.

As the antibodies which recognize the matrix protein of hMPV in the invention, antibodies No. 1 to No. 8 produced by the method described below were used. As a Comparative Example, the following commercial antibodies which recognize the N protein of hMPV were used.

### (Antibodies Recognizing N protein of hMPV)

- HMPV57 (manufactured by BIO-RAD): referred to as No. 9
- HMPV33 (manufactured by BIO-RAD): referred to as No. 10
- C01851M (manufactured by Meridian Life Science, Inc.): referred to as No. 11

### (Production of Antibodies in the Invention) Production Example 1

Antibodies which recognize the matrix protein of hMPV were produced as follows. First, a peptide having the amino acid sequence of the matrix protein of hMPV of SEQ ID NO: 1 was synthesized. A His-tag expression vector, pET302/NT-His, was cut with a restriction enzyme, *Eco*RI*,* then treated with alkaline phosphatase as dephosphorylation treatment and mixed with the peptide, and ligation reaction was caused using DNA Ligation Kit Ver. 2 (Takara Bio Inc.).

The recombinant matrix protein plasmid to which the target gene had been incorporated was introduced into a recombinant protein expression host, *E. coli* BL(DE3)pLysS (Novagen). The transformed bacterium was cultured on an LB agar plate, and a colony obtained was cultured with LB liquid medium. The expression of the recombinant matrix protein was induced by adding 1 mM IPTG (Takara Bio Inc.), and then *E. coli* was collected. The collected bacterium was suspended again in a solubilization buffer [0.5% Triron X-100 (sigma), 10 mM imidazole, 20 mM phosphate and 0.5 M NaCl (pH 7.4) (Amersham)] and solubilized by ultrasonic treatment, and then the recombinant hMPV matrix protein was purified using His trap Kit (Amersham). The purified protein was dialyzed using phosphate-buffered saline (referred to as PBS below), and the target recombinant hMPV matrix protein was thus obtained.

Monoclonal antibodies to the recombinant hMPV matrix protein were produced using the obtained recombinant hMPV matrix protein as the antigen for immunization. The monoclonal antibodies were produced as follows according to a general method. The recombinant hMPV matrix protein in an amount of 100 µg and an equivalent amount of Aduvant Complete Freund (Difco) were mixed, and a mouse (BALB/c, five weeks old, Japan SLC, Inc.) was immunized three times. The spleen cells were used for cell fusion. Mouse myeloma cells, Sp2/0-Ag14 cells (Shulman *et al.*, 1978) were used for the cell fusion. A culture solution obtained by adding 0.3 mg/ml L-glutamine, 100 U/ml penicillin G potassium, 100 µg/ml streptomycin sulfate and 40 µg/ml Gentacin to Dulbecco's Modified Eagle Medium (Gibco) (DMEM) and further adding fetal bovine serum (JRH) at 10% was used for culturing the cells. The cells were fused by mixing the spleen cells of the immunized mouse and Sp2/0-Ag14 cells and adding polyethylene glycol solution (Sigma) thereto. The fused cells were cultured in HAT-DMEM [serum-containing DMEM containing 0.1 mM sodium hypoxantine, 0.4 µM aminopterin and 0.016 mM thymidine (Gibco)], and the production of antibodies in the culture supernatant was confirmed by enzyme-linked immunosorbent assay (ELISA). Antibody production-positive cells were cultured in HT-DMEM [serum-containing DMEM containing 0.1 mM sodium hypoxantine and 0.16 mM thymidine] and further cultured in serum-containing DMEM.

The cloned cells were injected into the abdominal cavities of mice (BALB/c, retired, Japan SLC, Inc.) to which 2,6,10,14-tetramethylpentadecane (Sigma) had been injected, and the ascites were collected. The ascites were subjected to a protein G column, and monoclonal antibodies were purified.

The monoclonal antibodies thus obtained were screened by direct ELISA method using a 96-well plate in which the recombinant hMPV matrix protein was immobilized. As a result, eight kinds of antibody recognizing hMPV matrix protein were obtained. The eight kinds of antibody are called antibodies of No. 1 to No. 8 in the following explanation.

### Reference Example 1 (ELISA Test)

Next, the amino acid sequences which the antibodies produced above recognize were examined by an ELISA test.

The amino acid sequence of hMPV matrix protein of SEQ ID NO: 1 were divided into the six amino acid sequences below, and peptides having the respective amino acid sequences were produced by solid-phase peptide synthesis, which is a general method for chemically synthesizing a peptide. In this regard, peptide 1 corresponds to the amino acid sequence of the 6-30th residues (SEQ ID NO: 3) of hMPV matrix protein of SEQ ID NO: 1, and peptide 2 corresponds to the 40-70th residues (SEQ ID NO: 4). Peptide 3 corresponds to the 75-109th residues (SEQ ID NO: 5), and peptide 4 corresponds to the 123-141st residues (SEQ ID NO: 6). Peptide 5 corresponds to the 149-178th residues (SEQ ID NO: 2), and peptide 6 corresponds to the 184-219th residues (SEQ ID NO: 7).
Peptide 1: VDTYQGIPYTAAVQVDLVEKDLLPA (SEQ ID NO: 3)
Peptide 2: QANTPPAVLLDQLKTLTITTLYAASQNGPIL (SEQ ID NO: 4)
Peptide 3: SAQGAAMSVLPKKFEVNATVALDEYSKLDFDKLTV (SEQ ID NO: 5)
Peptide 4: YGMVSKFVSSAKSVGKKTH (SEQ ID NO: 6)
Peptide 5: FMDLEKNIPVTIPAFIKSVSIKESESATVE (SEQ ID NO: 2)
Peptide 6: EADQALTQAKIAPYAGLIMIMTMNNPKGIFKKLGAG (SEQ ID NO: 7)

First, 0.5 µg/well of the peptides (peptides 1 to 6) as the antigens and 0.1 µg/well of Recombinant Matrix Protein as a control were dispensed to a Nunc Immuno modules (manufactured by Thermo Fisher Scientific, code 469949) 96-well plate for ELISA, and the plate was maintained at 4°C overnight. Moreover, blank wells to which no antigen were dispensed were also prepared. Then, the wells were washed three times with 300 µL of PBST (0.05% Tween 20 in PBS), and the liquids remaining in the wells were removed by hitting the plate onto a paper towel. As a blocking solution, 300 µL of an Na caseinate (manufactured by Wako Pure Chemical Industries, Ltd.) solution was added, and the plate was incubated at 37°C for an hour. Then, the Na caseinate solution was removed. The wells were washed three times with 300 µL of PBST (0.05% Tween 20 in PBS), and the liquids remaining in the wells were removed by hitting the plate onto a paper towel. In this manner, the peptides were immobilized in the wells.

As primary antibodies, 100 µL of the antibodies of No. 1 to No. 8 in 1 µg/mL in 1% BSA-PBS blocking solution (primary antibody solutions) were added to the wells, and the plate was incubated at 37°C for an hour. Then, the primary antibody solutions were removed, and the wells were washed three times with 300 µL of PBST (0.05% Tween 20 in PBS).

As the secondary antibody, 100 µL of 10 µg/mL Anti Mouse IgG (H+L), Rabbit, IgG Whole, Peroxidase Cojugated (manufactured by Wako Pure Chemical Industries, Ltd., code 014-17611) was added to the wells, and the plate was incubated at 37°C for 1.5 hours. Then, the BSA solution was removed. The wells were washed three times with 300 µL of PBST (0.05% Tween 20 in PBS), and the liquids remaining in the wells were removed by hitting the plate onto a paper towel.

Sure Blue Reserve TMB Microwell Peroxidase Substrate (1-Component) (manufactured by KPL, code 53-00-01) in a volume of 100 µL was added to the wells as a chromogenic substrate, and the reaction was advanced for 15 minutes. The reaction was stopped by adding 100 µL of 2N sulfuric acid. Then, the absorbances at 450 nm were measured using a microplate reader (manufactured by BIO-RAD).

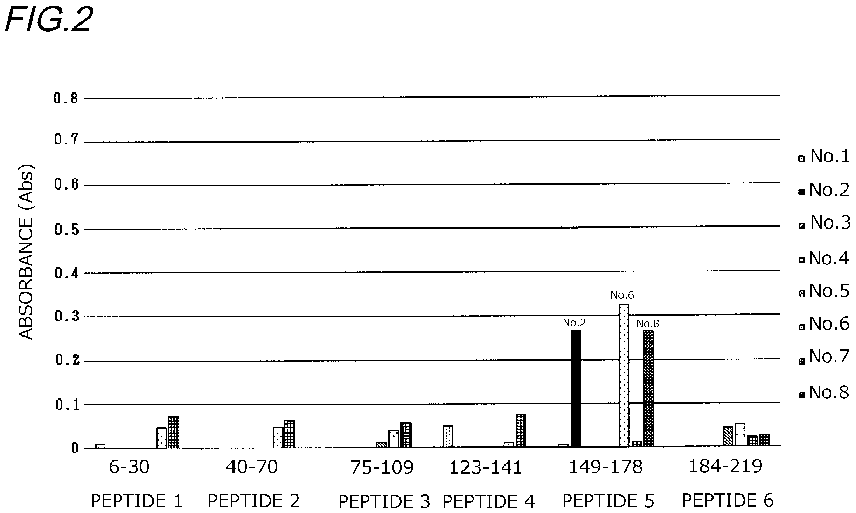

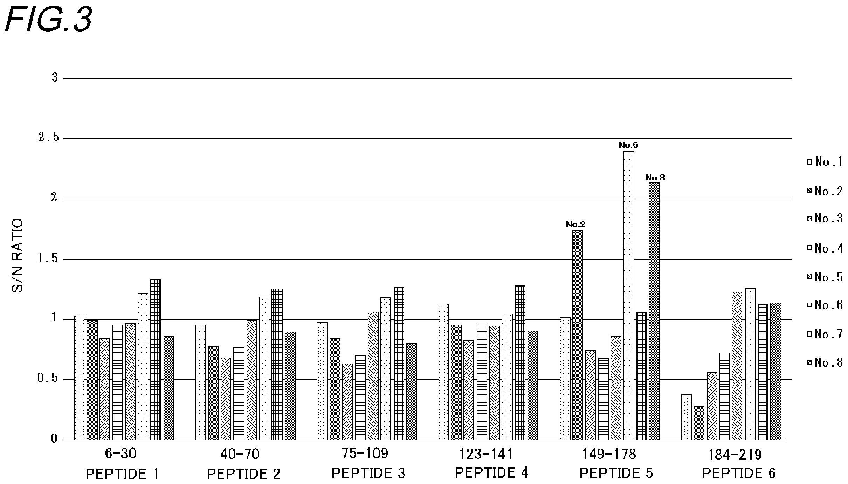

The values (the measurement values) obtained by subtracting the absorbance of the blank (the blank value) from the absorbances (the actual values) of the wells are shown in Table 1 and Fig. 2. Moreover, the values (the S/N ratios) obtained by dividing the absorbances (the actual values) of the wells by the absorbance of the blank (the blank value) are shown in Table 2 and Fig. 3.

**[Table 1]**

| | | | | | | | | |
|---|---|---|---|---|---|---|---|---|
| Table 1 | | | | | | | | |

| | Antibody | | | | | | | |
|---|---|---|---|---|---|---|---|---|
| | No. 1 | No. 2 | No. 3 | No. 4 | No. 5 | No. 6 | No. 7 | No. 8 |
| Peptide 1 (6-30th residues) | 0.009 | -0.004 | -0.054 | -0.015 | -0.008 | 0.047 | 0.072 | -0.03 |
| Peptide 2 (40-70th residues) | -0.019 | -0.097 | -0.136 | -0.1 | -0.003 | 0.048 | 0.064 | -0.028 |
| Peptide 3 (75-109th residues) | -0.012 | -0.062 | -0.145 | -0.119 | 0.013 | 0.04 | 0.058 | -0.045 |
| Peptide 4 (123-141st residues) | 0.05 | -0.018 | -0.071 | -0.019 | -0.016 | 0.012 | 0.075 | -0.026 |
| Peptide 5 (149-178th residues) | 0.006 | 0.267 | -0.095 | -0.119 | -0.033 | 0.325 | 0.013 | 0.265 |
| Peptide 6 (184-219th residues) | -0.022 | -0.111 | -0.172 | -0.111 | 0.045 | 0.052 | 0.024 | 0.028 |
| Recomb. MP | 0.691 | 3.444 | 2.326 | 0.236 | 0.496 | 3.457 | 0.58 | 3.484 |

**[Table 2]**

| | | | | | | | | |
|---|---|---|---|---|---|---|---|---|
| Table 2 | | | | | | | | |

| | Antibody | | | | | | | |
|---|---|---|---|---|---|---|---|---|
| | No. 1 | No. 2 | No. 3 | No. 4 | No. 5 | No. 6 | No. 7 | No. 8 |
| Peptide 1 (6-30th residues) | 1.027 | 0.988 | 0.836 | 0.955 | 0.963 | 1.215 | 1.329 | 0.863 |
| Peptide 2 (40-70th residues) | 0.955 | 0.772 | 0.681 | 0.765 | 0.988 | 1.186 | 1.248 | 0.891 |
| Peptide 3 (75-109th residues) | 0.969 | 0.841 | 0.628 | 0.695 | 1.058 | 1.179 | 1.260 | 0.798 |
| Peptide 4 (123-141st residues) | 1.124 | 0.955 | 0.824 | 0.953 | 0.941 | 1.044 | 1.278 | 0.904 |
| Peptide 5 (149-178th residues) | 1.017 | 1.736 | 0.738 | 0.672 | 0.858 | 2.395 | 1.056 | 2.137 |
| Peptide 6 (184-219th residues) | 0.369 | 0.280 | 0.560 | 0.716 | 1.221 | 1.255 | 1.118 | 1.137 |
| Recomb. MP | 5.188 | 21.87 | 15.10 | 2.430 | 4.006 | 21.95 | 4.515 | 22.12 |

As shown in Table 1 and Fig. 2, the absorbances of No. 2, No. 6 and No. 8 were 0.1 Abs or more when reacted with peptide 5. Moreover, as shown in Table 2 and Fig. 3, the S/N ratios of the antibodies were 1.5 or more when reacted with peptide 5. That is, No. 2, No. 6 and No. 8 substantially exhibited the antigen-antibody reaction with peptide 5.

From the above results, it was found that the antibodies of No. 2, No. 6 and No. 8 are antibodies which recognize the amino acid sequence of the 149-178th residues of hMPV matrix protein having the amino acid sequence of SEQ ID NO: 1.

### Reference Example 2 (SDS-PAGE and Western Blotting)

Next, whether the antibodies of No. 1 to No. 8 produced above recognize the three-dimensional structure of hMPV matrix protein was examined by SDS-PAGE and western blotting.

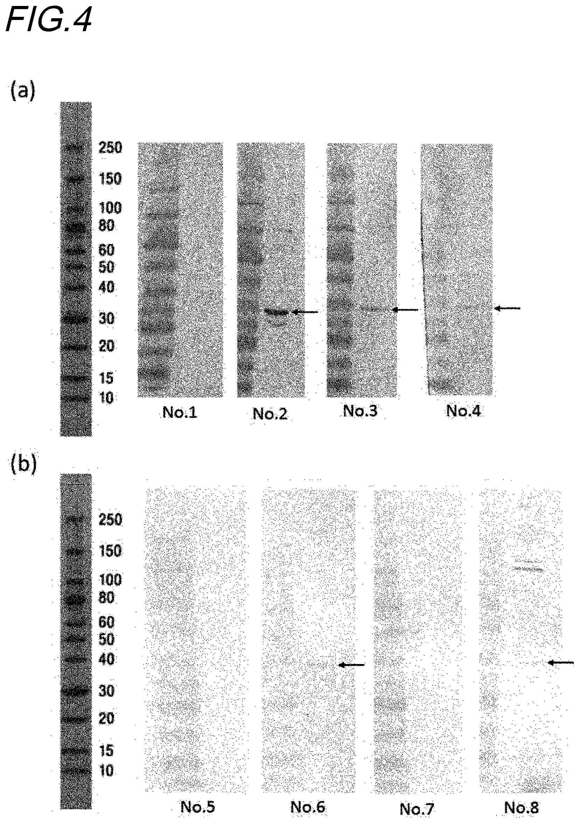

A 0.01 mg/ml Recombinant hMPV Matrix Protein solution was mixed with an equivalent amount of 2× sample buffer (125 mM Tris-HCl, 10% glycerol, 4% SDS, 3% TCEP, 0.001% bromophenol blue, pH 6.8) and subjected to SDS-PAGE. The SDS-PAGE was conducted using XV PANTERA Gel 5-20% 18well (manufactured by DRC) according to a known standard method. The protein was transferred from the gel after the electrophoresis to Sequi-Blot PVDF Membranes (manufactured by BIO-RAD) using a blotting apparatus (manufactured by BIO-RAD). The PVDF membranes after the transfer were subjected to blocking with 1% BSA-PBS at room temperature for 10 minutes. The blocking solution was removed, and the PVDF membranes were washed three times with PBS containing 0.05% Tween 20 (product name) (referred to as T-PBS below) for 10 minutes and then incubated with the antibodies of No. 1 to No. 8 at room temperature for an hour. The PVDF membranes were washed three times with T-PBS for 10 minutes and then incubated with an alkaline phosphatase-labeled anti-mouse IgG (manufactured by SIGMA) which had been diluted 5000 times with T-PBS at room temperature for 30 minutes. After washing three times with T-PBS for 10 minutes, the PVDF membranes were incubated with 1-Step^{™} NBT/BCIP (manufactured by PIERCE), which is a chromogenic substrate, and the antibodies bound to the PVDF membranes were visualized. The results are shown in Fig. 4.

As shown in Fig. 4, bands of about 36 kDa, which correspond to the whole length hMPV matrix protein, were detected when the antibodies of No. 2, No. 3, No. 4, No. 6 and No. 8 were used while the band was not detected when the antibodies of No. 1, No. 5 and No. 7 were used.

That is, the antibodies of No. 1, No. 5 and No. 7 did not undergo the antigen-antibody reaction with the whole length matrix protein of hMPV separated by SDS-PAGE by western blotting, and thus it was found that the antibodies recognize the three-dimensional structure of hMPV matrix protein.

### <Example 1>

Immunochromatography analysis kits including an analyte dilution solution and an immunochromatography analysis device including a sample application part 1, a labeling substance-holding part 2, a chromatography medium part 3 having a detection part 4 and an absorption part 5 were produced.

The antibodies of No. 1 to No. 3 produced above were used for the labeled antibody contained in the labeling substance-holding part and the detection antibody contained in the detection part, and the combinations are as shown in Table 3 below. The details are explained below.

### (1) Production of Sample Application Part

A nonwoven cloth composed of glass fibers (manufactured by Millipore Corporation: 300 mm × 30 mm) was used as the sample application part.

### (2) Production of Labeling Substance-Holding Part

To 0.5 ml of a colloidal gold suspension (manufactured by Tanaka Kikinzoku Kogyo K.K.: LC 40 nm), 0.1 ml of an antibody shown in Table 3 (labeled antibody) which had been diluted to a concentration of 0.05 mg/ml with a phosphate buffer (pH 7.4) was added, and the mixture was left to stand still at room temperature for 10 minutes.

Next, 0.1 ml of a phosphate buffer (pH 7.4) containing 1 mass% bovine serum albumin (BSA) was added, and the mixture was further left to stand still at room temperature for 10 minutes. Then, after stirring thoroughly, the mixture was centrifuged at 8000× g for 15 minutes, and the supernatant was removed. Then, 0.1 ml of a phosphate buffer (pH 7.4) containing 1 mass% BSA was added. A labeling substance solution was produced by the above procedures.

A solution obtained by adding 300 µL of a 10 mass% aqueous trehalose solution and 1.8 mL of distilled water to 300 µL of the labeling substance solution produced above was evenly applied to a 12 mm × 300 mm glass fiber pad (manufactured by Millipore Corporation) and then dried with a vacuum dryer, and the labeling substance-holding part was thus produced. The amount of the labeled antibody contained in the labeling substance-holding part was adjusted to 0.07 µg/device (0.2 µg/cm²).

### (3) Production of Chromatography Medium Part and Detection Part

A sheet composed of nitrocellulose (manufactured by Millipore Corporation, product name: HF120, 300 mm × 25 mm) was used as a membrane. Next, 150 µL of a solution obtained by diluting an antibody shown in Table 3 (detection antibody) to a concentration of 1.0 mg/ml with a phosphate buffer (pH 7.4) containing 5 mass% isopropyl alcohol was applied to a detection part on the dried membrane in a line with a width of 1 mm using a dispenser for immunochromatography "XYZ3050" (manufactured by BIODOT) at an amount of 1 µL/mm (25 µL per sheet).

Moreover, to check whether the gold nanoparticle labeling substance has been developed or not and to check the development speed, a solution obtained by diluting goat-derived antiserum having a broad affinity range with the gold nanoparticle labeling substance with a phosphate buffer (pH 7.4) was applied to a control part (a control line) in the downstream of the detection part. Then, by drying at 50°C for 30 minutes and drying at room temperature overnight, the chromatography medium part and the detection part were produced. The amount of the detection antibody contained in the detection part was adjusted to 0.15 µg/device (1.5 µg/cm²).

### (4) Production of Immunochromatography Analysis Devices

Next, the sample application part, the labeling substance-holding part, the chromatography medium part having the detection part and a nonwoven cloth made of glass fibers as an absorption part for absorbing the developed sample and the labeling substance were attached one by one to a base material composed of a backing sheet. Then, the obtained product was cut with a width of 5 mm with a cutter, and the immunochromatography analysis devices were thus obtained. The length of the labeling substance-holding part in the direction of sample development was adjusted to 12 mm.

### (5) Preparation of Analyte Dilution Solution

A 50 mM HEPES buffer (pH 7.5) containing 1 mass% nonionic surfactant (a 1:1 mixture of Nonidet P-40 manufactured by Nacalai Tesque, Inc. and Nonion MN-811 manufactured by NOF Corporation) was prepared and used as the analyte dilution solution for diluting an analyte.

### <Example 2>

Immunochromatography analysis kits of Example 2 were produced in the same manner as in Example 1 except that the antibodies of No. 5 to No. 8 produced above were used as the labeled antibody contained in the labeling substance-holding part and the detection antibody contained in the detection part in the combinations shown in Table 4 in Example 1.

### <Comparative Example 1>

Immunochromatography analysis kits of Comparative Example 1 were produced in the same manner as in Example 1 except that the antibodies of No. 9 to No. 11, which are antibodies that recognize the N protein of hMPV, were used as the labeled antibody contained in the labeling substance-holding part and the detection antibody contained in the detection part in the combinations shown in Table 5 in Example 1.

### Test Example 1 (Evaluation of Detection Sensitivity)

The color intensities of the detection parts were measured as follows using the immunochromatography analysis devices of Examples 1 and 2 and Comparative Example 1 produced above using an analyte sample containing hMPV.

A nasal aspirate of a person infected with hMPV was diluted 5000 times with the analyte dilution solution, and an analyte-containing solution was thus prepared. The prepared analyte-containing solution in a volume of 120 µL was put on the sample application parts of the immunochromatography analysis devices and developed. The degrees of coloration (color intensities) of the detection parts were measured with a densitometer five minutes after starting the development.

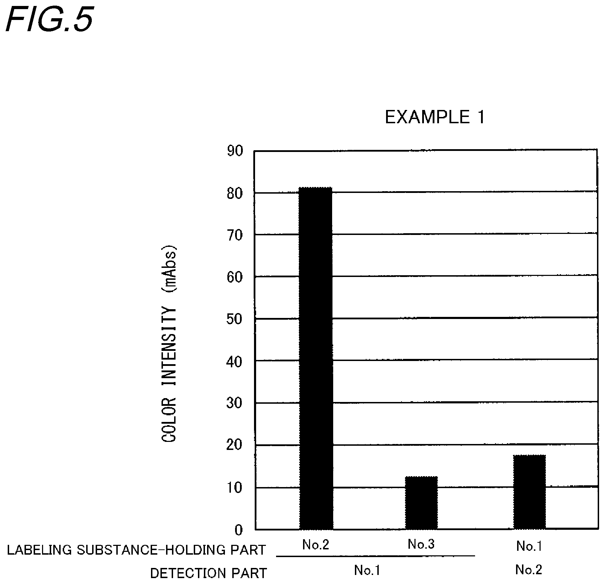

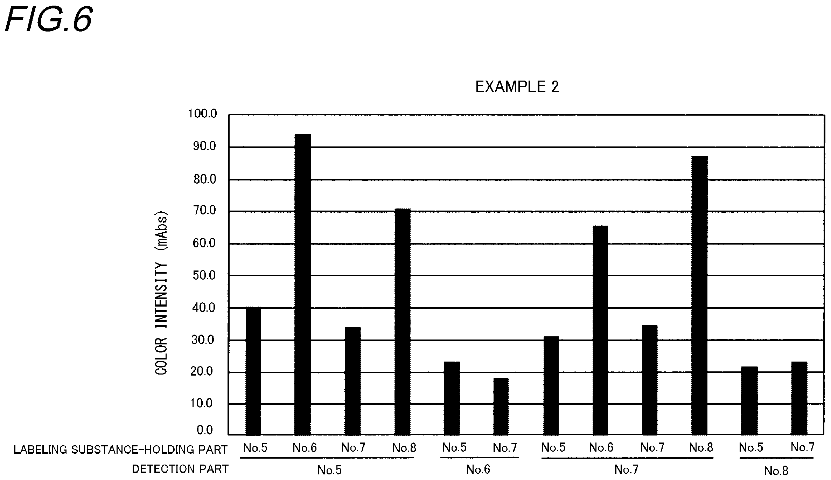

The results of Example 1 are shown in Table 3 and Fig. 5, and the results of Example 2 are shown in Table 4 and Fig. 6. The results of Comparative Example 1 are shown in Table 5.

**[Table 3]**

| | | | |
|---|---|---|---|
| Table 3 Example 1 | | | |

| Detection Part | No. 1 | | No. 2 |
|---|---|---|---|
| Labeling Substance-Holding Part | No. 2 | No. 3 | No. 1 |
| Actual Analyte (×5000 Dilution) | 81.3 | 12.5 | 17.6 |

| | | | |
|---|---|---|---|
| No. 1: Antibody recognizing the three-dimensional structure of the matrix protein of hMPV No. 2: Antibody recognizing the amino acid sequence of 149-178th residues of the matrix protein of hMPV. | | | |

**[Table 4]**

| | | | | | | | | | | | | |
|---|---|---|---|---|---|---|---|---|---|---|---|---|
| Table 4 Example 2 | | | | | | | | | | | | |

| Detection Part | No. 5 | | | | No. 6 | | No. 7 | | | | No. 8 | |
|---|---|---|---|---|---|---|---|---|---|---|---|---|
| Labeling Substance-Holding Part | No. 5 | No. 6 | No. 7 | No. 8 | No. 5 | No. 7 | No. 5 | No. 6 | No. 7 | No. 8 | No. 5 | No. 7 |
| Actual Analyte (×5000 Dilution) | 40.5 | 94.2 | 34.0 | 70.8 | 23.1 | 18.1 | 31.2 | 65.7 | 34.6 | 87.2 | 21.7 | 23.2 |

| | | | | | | | | | | | | |
|---|---|---|---|---|---|---|---|---|---|---|---|---|
| No. 5 and 7: Antibodies recognizing the three-dimensional structure of the matrix protein of hMPV No. 6 and 8: Antibodies recognizing the amino acid sequence of 149-178th residues of the matrix protein of hMPV | | | | | | | | | | | | |

**[Table 5]**

| | | | | | | | | | |
|---|---|---|---|---|---|---|---|---|---|
| Table 5 Comparative Example 1 | | | | | | | | | |

| Detection Part | No. 9 | | | No. 10 | | | No. 11 | | |
|---|---|---|---|---|---|---|---|---|---|
| Labeling Substance-Holding Part | No. 9 | No. 10 | No. 11 | No. 9 | No. 10 | No. 11 | No. 9 | No. 10 | No. 11 |
| Actual Analyte (×5000 Dilution) | 0.0 | 0.0 | 0.0 | 0.0 | 3.2 | 0.0 | 0.0 | 0.0 | 0.0 |

As seen from the above results, Examples 1 and 2, in which antibodies that recognize the matrix protein of hMPV were used, hMPV could be detected from the actual analyte of hMPV with a high sensitivity as compared to the cases using antibodies that recognize the N protein of hMPV.

In particular, hMPV could be detected with a particularly excellent sensitivity in immunochromatography assay when antibodies which recognize the amino acid sequence of the 149-178th residues of hMPV matrix protein having the amino acid sequence of SEQ ID NO: 1 were used as the labeled antibody and when antibodies which recognize the three-dimensional structure of hMPV matrix protein were used as the detection antibody.

On the other hand, in Comparative Example 1, in which antibodies that recognize the N protein of hMPV (No. 9 to No. 11) were used, the actual analyte of hMPV could not be detected.

### Test Example 2 (Evaluation of Cross-Reactivity)

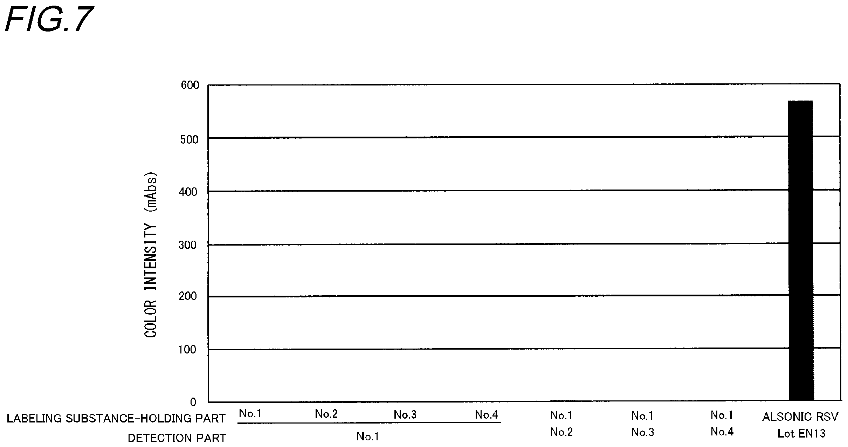

In this test, measurement was conducted using the immunochromatography analysis kits of Example 1 produced above and using RSV (product name Respiratory Syncytial Virus, manufactured by Microbix Biosystem, model number EL-07-02) as the analyte. An analyte-containing solution was prepared by diluting with the analyte dilution solution in a manner that the RSV concentration became 35 µg/mL, and the measurement was conducted in the same manner as in Test Example 1 using the analyte-containing solution as the sample. Moreover, the same test was conducted using an RSV detection kit (product name: Alsonic RSV, Lot. EN13, manufactured by Alfresa Pharma Corporation) as a positive control. The results are shown in Table 6 and Fig. 7.

**[Table 6]**

| | | | | | | | | |
|---|---|---|---|---|---|---|---|---|
| Table 6 | | | | | | | | |

| Detection Part | No. 1 | | | | No. 2 | No. 3 | No. 4 | |
|---|---|---|---|---|---|---|---|---|
| Labeling Sub stance-Holding Part | No. 1 | No. 2 | No. 3 | No. 4 | No. 1 | No. 1 | No. 1 | Alsonic RSV Lot. EN13 |
| RSV | 0 | 0 | 0 | 0 | 2.9 | 0 | 0 | 567 |

As shown in Table 6 and Fig. 7, it was found that the immunochromatography analysis kits of the invention can specifically detect hMPV without causing a cross-reaction with RSV, which has a high homology to hMPV.

Although the invention has been explained in detail referring to specific embodiments, it is obvious to one skilled in the art that various changes and modifications can be made without departing from the spirit and the scope of the invention. The present application is based on a Japanese patent application filed on March 26, 2019 (patent application No. 2019-058483), which is hereby incorporated by reference in its entirety. All the references cited here are incorporated in their entirety.

### REFERENCE SIGNS LIST

- 1.: Sample application part
- 2.: Labeling substance-holding part
- 3.: Chromatography medium part
- 4.: Detection part
- 5.: Absorption part
- 6.: Backing sheet

## Claims

1. A human metapneumovirus detection reagent **characterized by** containing an antibody which recognizes the matrix protein of human metapneumovirus.

2. The human metapneumovirus detection reagent according to claim 1, wherein the antibody recognizes the amino acid sequence of the 149-178th residues of the matrix protein of human metapneumovirus having the amino acid sequence of SEQ ID NO: 1.

3. An immunological assay for human metapneumovirus including detecting human metapneumovirus in an analyte with an antibody which recognizes the matrix protein of human metapneumovirus.

4. The immunological assay according to claim 3, wherein the immunological assay is enzyme immunoassay, agglutination assay or immunochromatography assay.

5. The immunological assay according to claim 4,
wherein the immunological assay is immunochromatography assay,
wherein the immunochromatography assay includes
a step of bringing a labeled antibody in which a labeling substance is bound to an antibody which recognizes the matrix protein of human metapneumovirus into contact with the human metapneumovirus in the analyte and thus forming a complex of the labeled antibody and the human metapneumovirus, and
a step of detecting the human metapneumovirus in the complex with a detection antibody which recognizes the matrix protein of human metapneumovirus.

6. The immunological assay according to claim 5, wherein the labeled antibody recognizes the amino acid sequence of the 149-178th residues of the matrix protein of human metapneumovirus having the amino acid sequence of SEQ ID NO: 1.

7. The immunological assay according to claim 5 or 6, wherein the detection antibody recognizes the three-dimensional structure of the matrix protein of human metapneumovirus.

8. An immunochromatography analysis device for detecting human metapneumovirus including a sample application part, a labeling substance-holding part, a chromatography medium part having a detection part and an absorption part,
wherein the labeling substance-holding part and the detection part contain an antibody which recognizes the matrix protein of human metapneumovirus.

9. The immunochromatography analysis device according to claim 8, wherein the antibody contained in the labeling substance-holding part recognizes the amino acid sequence of the 149-178th residues of the matrix protein of human metapneumovirus having the amino acid sequence of SEQ ID NO: 1.

10. The immunochromatography analysis device according to claim 8 or 9, wherein the antibody contained in the detection part recognizes the three-dimensional structure of the matrix protein of human metapneumovirus.

11. An immunochromatography analysis kit including the immunochromatography analysis device according to any one of claims 8 to 10 and an analyte dilution solution for diluting an analyte.
